# EUROPEAN PATENT SPECIFICATION

(11) **EP 3 009 839 B1**
(45) Date of publication and mention of the grant of the patent: **28.11.2018**
(21) Application number: 14810344.3
(22) Date of filing: 29.04.2014
(51) Int. Cl.: G01N 33/53, G01N 21/76, G01N 30/72

(54) **METHOD FOR DETECTING HYPOXIA OR DIAGNOSING HYPOXIA-RELATED DISEASES**
VERFAHREN ZUR ERKENNUNG VON HYPOXIE ODER ZUR DIAGNOSE VON HYPOXIE-VERMITTELTEN ERKRANKUNGEN
PROCÉDÉ POUR DÉTECTER UNE HYPOXIE OU DIAGNOSTIQUER DES MALADIES LIÉES À UNE HYPOXIE

(30) Priority: 14.06.2013 KR 20130068324; 12.07.2013 KR 20130082024
(43) Date of publication of application: 20.04.2016
(73) Proprietor: Seoul National University R&DB Foundation, Gwanak-gu Seoul 08826 (KR)
(72) Inventor: PARK, Jong Wan, Seoul 137-950 (KR); SHIN, Hyun Woo, Paju-si Gyeonggi-do 413-100 (KR); CHO, Joo-Youn, Seoul 137-764 (KR); RHEE, Chae-Seo, Yongin-si Gyeonggi-do 448-160 (KR); HONG, Il-Hee, Seoul 135-794 (KR)
(74) Representative: Ter Meer Steinmeister & Partner
(86) International application number: PCT/KR2014/003770
(87) International publication number: WO 2014/200178

(56) References cited:
- EP-A1- 2 592 420
- WO-A1-03/017996
- WO-A2-2008/054208
- WO-A2-2009/063226
- US-A1- 2010 233 746
- US-A1- 2012 136 581
- US-A1- 2012 136 581
- GIOVANNA E CARPAGNANO ET AL: "Non-invasive study of airways inflammation in sleep apnea patients", SLEEP MEDICINE REVIEWS, W.B. SAUNDERS, AMSTERDAM, NL, vol. 15, no. 5, 28 December 2010 (2010-12-28), pages 317-326, XP028264267, ISSN: 1087-0792, DOI: 10.1016/J.SMRV.2010.12.005 [retrieved on 2011-01-08]
- Christina Vafeas ET AL: "Hypoxia Stimulates the Synthesis of Cytochrome P450-Derived Inflammatory Eicosanoids in Rabbit Corneal Epithelium 1", , 1 January 1998 (1998-01-01), XP055336482, Retrieved from the Internet: URL:http://jpet.aspetjournals.org/content/ 287/3/903.full.pdf [retrieved on 2017-01-18]
- BASIL O. IBE ET AL: "Endogenous Arachidonic Acid Metabolism by Calcium Ionophore A23187-stimulated Lamb Lungs: Effect of Hypoxia", AMERICAN JOURNAL OF RESPIRATORY CELL AND MOLECULAR BIOLOGY., vol. 4, no. 4, 1 April 1991 (1991-04-01), pages 379-385, XP055336480, NEW YORK, NY, US ISSN: 1044-1549, DOI: 10.1165/ajrcmb/4.4.379
- RAMI KHAYAT ET AL: "Obstructive sleep apnea: the new cardiovascular disease. Part I: obstructive sleep apnea and the pathogenesis of vascular disease", HEART FAILURE REVIEWS, KLUWER ACADEMIC PUBLISHERS, BO, vol. 14, no. 3, 20 September 2008 (2008-09-20), pages 143-153, XP019676658, ISSN: 1573-7322
- SHIN H ET AL: "Hypoxia-inducible 5-eicosatetraenoates are potential markers for diagnosing obstructive sleep apnea", SLEEP MEDICINE, vol. 16, March 2015 (2015-03), XP029353007, ISSN: 1389-9457, DOI: 10.1016/J.SLEEP.2015.02.505
- YANG R K ET AL: "Relationship between 3-Methoxy-4-Hydroxyphenylglycol and Homovanillic Acid in Saliva and Plasma of Healthy Volunteers", BIOLOGICAL PSYCHIATRY, ELSEVIER SCIENCE, NEW YORK, NY; US, vol. 42, no. 9, 1 November 1997 (1997-11-01), pages 821-826, XP027446826, ISSN: 0006-3223, DOI: 10.1016/S0006-3223(97)00055-3 [retrieved on 1997-11-01]

## Description

### BACKGROUND OF THE INVENTION

### Field of the Invention

The present disclosure relates to a field of detecting hypoxia or diagnosing hypoxia-related diseases.

### Description of the Related Art

Hypoxia is generally regarded as a pathological status in which oxygen required to sustain a human life is insufficiently supplied. It has been known that systemic hypoxia is as a fundamental cause of various diseases, such as, obstructive sleep apnea, asthma, chronic obstructive pulmonary disease (COPD, Lung fibrosis), pulmonary hypertension and pulmonary edema, pulmonary thromboembolism, cardiac failure, hypoxic ischemic encephalopathy, and perinatal asphyxia (Savransky et al. Am J Respir Crit Care Med. 2007; 175 (12):1290-7). Further, local hypoxia is a central pathophysiological factor of major detrimental diseases, such as, cerebrovascular diseases, cardiovascular diseases, and tumors. A living body may be frequently exposed to a hypoxia status due to various causes, such as, environmental changes (an alpine region, and the like) or respiratory diseases, but the homeostasis of oxygen is finely adjusted or controlled by various compensation mechanisms at an individual as a whole and tissue levels.

For such hypoxia-related diseases, determining the exact condition or extent of hypoxia under which a body as a whole or a specific organ has been exposed is a key to understand the cause of the diseases and diagnose them. However, up to now, diagnosis of hypoxia stays at the level of measuring oxygen saturation level in the blood at a particular moment. Optical oxygen saturation meters are widely used for non-invasive determination of oxygen concentration of the blood and measure oxygen saturation levels based on the difference in the absorbance resulting from the differential degree of oxygen binding to hemoglobin. More invasive way is an arterial blood gas analysis test in which the amount of oxygen, carbon dioxide and the like in the blood is measured using an arterial blood from a patient. The two methods described just estimate the oxygen level in the blood at a particular moment and do not provide any information regarding a hypoxic state a person has been exposed over a period of time. Further, in the conventional methods, the meter needs to be attached to the patient or invasive process such as arteriopuncture is often inevitable. Accordingly, there are needs to develop an improved assessment method of chronic hypoxia, which is crucial for accurate understanding of the pathological physiology of hypoxia-related diseases, and also for diagnosis, monitor and/or prevention of hypoxia-related diseases enabling correlation analysis between the severity of the disease and the extent or degree of exposure to a hypoxia condition.

It has been known that a hypoxia inducible factor (HIF) known for inducing hypoxia is a transcription factor regulating about 60 genes required for cells to adapt in a hypoxic condition and HIF target proteins are involved in biological processes such as angiogenesis and angiectasis, energy production, cell proliferation and survival, or cell migration, and the like (Brahimi-Horn MC, et al. J Mol Med. 2007;85(12):1301-7; Wykoff et al. Cancer Res. 2000;60(24):7075-83; Park JW et al. J Pharmacol Sci. 2004 ;94(3):221-32; and Gort et al. Curr Mol Med. 2008 ;8(1):60-7).

U.S. Patent Publication No. 2004-0265926 relates to bodily fluid markers of tissue hypoxia and discloses an oxygen related protein 150 (ORP 150) which is a marker capable of detecting hypoxia as one clinical symptom of a heart disease.

It is presumed that the metabolites cells produce and secrete during a hypoxic adaptation process may be significantly different in amounts and types from those secreted under a regular oxygen environment (Majmundar, et al. Mol Cell. 2010;40 (2):294-309), but there are no reports of such metabolites yet, and discovery of metabolite markers in relation to hypoxia is required.
US 2010/0233746 A1 relates to a method for determining the systemic metabolic status of an organism in relation to inflammation and oxidative stress using a biological sample, comprising detection and quantification of one or more derivatives of arachidonic acid, linoleic acid and/or docosahexaenoic acid.
GIOVANNA E CARPAGNANO ET AL, "Non-invasive study of airways inflammation in sleep apnea patients", SLEEP MEDICINE REVIEWS, W.B. SAUNDERS, AMSTERDAM, NL, vol. 15, no. 5, pages 317 - 326 reviews current knowledge on the non-invasive airway markers and their potential clinical applications in obstructive sleep apnea syndrome.

### SUMMARY OF THE INVENTION

In order to solve the conventional problems, the present disclosure is to provide biomarkers capable of detecting chronic intermittent hypoxia and a method of detecting or diagnosing chronic intermittent hypoxia using the same.

In one aspect, the present disclosure provides an *in vitro* method for detecting chronic intermittent hypoxia, the method comprising detecting an arachidonic acid derivative selected from a 5-hydroxy-6,8,11,14-eicosatetraenoic acid (5-HETE), or a 5-oxo-6,8,11,14-eicosatetraenoic acid (5-oxoETE) from a biological sample: and diagnosing the sample as chronic intermittent hypoxia when the amount of the arachidonic acid derivative in the biological sample is increased as compared to a control group, wherein the biological sample is urine.

Detection substances which may be included in the composition of the present disclosure are not limited as long as they are capable of specifically recognizing an arachidonic acid or a derivative thereof. Examples thereof include receptors, ligands, substrates, antibodies, antibody fragments, antibody mimetics, aptamers, avidity multimers, or peptide mimetices, but the present disclosure is not limited thereto. The substance according to the present disclosure may be detected with various methods suitable for the substance, and for example, may be detected with an antibody analysis, chemiluminescent assay, or liquid chromatography-mass spectrometry assay, but the present disclosure is not limited thereto.

In the method according to the present disclosure, the arachidonic acid derivative may be measured by any one or more methods of an antibody analysis, chemiluminescent assay, or liquid chromatography-mass spectrometry assay, but the present disclosure is not limited thereto.

In another embodiment, the present disclosure provides an arachidonic acid derivative selected from a 5-hydroxy-6,8,11,14-eicosatetraenoic acid (5-HETE), or a 5-oxo-6,8,11,14-eicosatetraenoic acid (5-oxoETE) for use in the *in vitro* detection of chronic intermittent hypoxia.

### ADVANTAGEOUS EFFECTS

According to the present disclosure, simple but accurate detection of chronic intermittent hypoxia is possible in a short period of time by detecting the metabolites of an arachidonic acid selected from a 5-hydroxy-6,8,11,14-eicosatetraenoic acid (5-HETE), or a 5-oxo-6,8,11,14-eicosatetraenoic acid (5-oxoETE) in a subject suspected of hypoxia-related disease or hypoxia. The present markers, and methods can be advantageously used to diagnose or early diagnose or detection of chronic intermittent hypoxia,. Further using the present disclosure, monitoring the prognosis or determining of the therapeutic efficacies of the treatment may also be made.

### BRIEF DESCRIPTION OF THE DRAWINGS

FIG. 1 is a schematic diagram showing a 5-lipoxygenase (5-LO) pathway and metabolites of an arachidonic acid.
FIG. 2 is a graph showing the arachidonic acid-derived metabolites which are increased under a low oxygen condition in a monocyte-derived THP-1 cell line. N, normoxia (21% O₂); H, hypoxia (1% O₂); ReOxy, reoxygenation. **P* < 0.05.
FIG. 3 is a result showing that a target metabolite is reduced when the hypoxia inducible factor is inhibited. N, normoxia (21% O₂); H, hypoxia (1% O₂); R, reoxygenation. **P* < 0.05, ***P* < 0.01.
FIG. 4 is a result showing the decrease in the target metabolites when administering an inhibitor of a hypoxia inducible factor. N, normoxia (21% O₂); H, hypoxia (1% O₂); R, reoxygenation. **P* < 0.05, ***P* < 0.01.
FIG. 5 shows the arachidonic acid-derived metabolites which are increased in a low oxygen condition in a human primary monocyte. N, normoxia (21% O₂); H, hypoxia (1% O₂); IH, intermittent hypoxia; R, reoxygenation. **P* < 0.05, ***P* < 0.01.
FIG. 6 is a result showing that a target metabolite is reduced when a hypoxia inducible factor is inhibited in a human primary monocyte. N, normoxia (21% O₂); H, hypoxia (1% O₂); IH, intermittent hypoxia; R, reoxygenation; siHl, silencing HIF-1alpha. **P* < 0.05, ** *P* <0.01.
FIG. 7 shows that the activity of glutathione peroxidase (GPX) transforming 5-HpETE into 5-HETE is increased in a hypoxic environment. N, normoxia (21% O₂); H, hypoxia (1% O₂); R, reoxygenation. * *P* <0.05.
FIG. 8 is a result showing that the arachidonic acid-derived metabolites are increased in a first morning urine from an obstructive sleep apnea (OSA) patient.
FIG. 9 is a graph showing a correlation between the arachidonic acid-derived metabolites which are increased in urine of the OSA patient and minimum oxygen saturation.
FIG. 10 is a graph showing a correlation between the metabolites which are increased in urine of the OSA patient and an apnea-hypopnea index (AHI) .
In the present figures, all the statistical analyses were performed by using an IBM SPSS 18 (SPSS, Inc, Chicago, Ill) statistical program and using a Mann-Whitney U test.

### DETAILED DESCRIPTION OF THE EMBODIMENTS

The present disclosure is based on the discovery of biomarkers which are specifically present or expressed in a hypoxia condition and their use.

In one aspect, the disclosure relates to a use of the present biomarkers for determining or detecting chronic intermittent hypoxia as specified in claim 1. Particularly, in one embodiment, the present disclosure relates to a method of detecting/diagnosing chronic intermittent hypoxia using the present makers in a subject who is a hypoxic patient or a suspected of hypoxia.

In the present disclosure, the term "diagnosis" as used herein refers to determining the disease or disorder susceptibility of a subject, determining whether a subject has a specific disease or disorder, determining the prognosis (for example, determining the status of the disease or the response to the treatments) of a subject who has specific disease or disorder, or therametrics(for example, monitoring the status of a subject to provide the information on the efficacy of treatment).

In the present disclosure the term "detection" refers to determine the presence or absence or the extent of hypoxia and may be used interchangeably with diagnosis. For example, in the cases where a disease itself such as obstructive sleep apnea induces hypoxia, the detection of hypoxia is equivalent to diagnosis of the disease, and determining the extent of hypoxia may also be used to determine the severity of the disease. In other cases where a disease, such as for example pulmonary hypertension and hypoxic ischemic encephalopathy, is a result of hypoxia, the detection of hypoxic status of a body or a particular organ may be used for preventing and early diagnosing the corresponding disease.

In the present disclosure, the term "biomarker or marker for diagnosing/detecting" refers to an agent that may discriminate a hypoxic sample or hypoxic status from a normal sample including samples undergone appropriate treatment and having normal characteristics. Also included are biomolecules such as lipids and glycolipids which are increased or decreased in a sample or a patient which suffers from the disease compared to normal samples. In the present disclosure, the biomarkers are selected from a 5-hydroxy-6,8,11,14-eicosatetraenoic acid (5-HETE), or a 5-oxo-6,8,11,14-eicosatetraenoic acid (5-oxoETE), the amount of which is increased in hypoxia patient or samples.

The biomarker according to the present disclosure may be used alone or in a combination. Further, the present marker may be used in combination with existing diagnosis methods in the related art. Those skilled in the art may be able to select a combination of markers meeting the desired sensitivity and specificity in view of the analysis using biological materials from normal subject and patient as described in the present disclosure.

In the present disclosure, the term hypoxia or hypoxic disease refers to a condition in which the body or a region of the body is deprived of adequate oxygen supply due to the reduced oxygen concentration in the blood or the increased amount of oxygen requirement by the tissue. The types include chronic intermittent or persistent hypoxia resulting from a reduced partial pressure of oxygen in the arterial blood, acute hypoxia, hypoxic hypoxia, anemic hypoxia, stagnant hypoxia, histotoxic hypoxia, and water-soluble hypoxia. In an exemplary embodiment of the present disclosure, the marker of the present disclosure may act as a cumulative marker reflecting the extent and the duration of hypoxia. In this respect, the present marker is used for diagnosing chronic intermittent.

In another aspect, the chronic intermittent hypoxia of the present disclosure encompasses systemic or local hypoxia. The systemic hypoxia is accompanied by various diseases such as obstructive sleep apnea, asthma, chronic obstructive pulmonary disease (COPD, Lung fibrosis), pulmonary hypertension and pulmonary edema, pulmonary thromboembolism, cardiac failure, airway obstruction, pneumothorax, perinatal asphyxia, anemia, hemoglobinopathy, carbon monoxide poisoning, and cyanide poisoning without being limited thereto. Further, the local hypoxia is accompanied by a cerebrovascular disease, a cardiovascular disease, a cancer, and an ischemic tissue damage including hypoxic ischemic encephalopathy without being limited thereto.

In an exemplary embodiment of the present disclosure, the hypoxia is the one accompanied by obstructive sleep apnea. The hypoxia associated with obstructive sleep apnea is characterized by repeated cycles of reduction and restoration of oxygen saturation level in the blood in which the oxygen saturation level is reduced to for example 60% to 90% (to the extent that requires an oxygen mask) of the normal level due to the obstruction of an upper airway during sleep, followed by the restoration of the oxygen level to normal due to a strong respiratory drive (Somers VK et al. J Clin Invest 1995; 96: 1897-904). In certain cases, the oxygen saturation is lowered to a level less than 60% and in this case, possibility of sudden death is high.

In the present disclosure, the obstructive sleep apnea frequently causes respiratory arrest during sleep and may be divided into obstructive sleep apnea and central sleep apnea. Most of sleep apnea patients have anatomically characteristic phenotype in which the upper airway space is narrow. In sleep apnea syndrome patients, fat is frequently accumulated around airways due to obesity, or the oropharyngeal soft tissues such as tongue and tonsil are larger in size and thus the upper airway becomes narrow than in healthy controls. Most of the patients suffer from obstructive sleep apnea in which the upper airway is closed and thus the patients are exposed to the systemic hypoxia during sleep. Main symptoms include chronic intermittent hypoxia in which the oxygen saturation level is reduced to for example 60% to 90% (to the extent that requires an oxygen mask) of the normal level due to the obstruction of an upper airway during sleep, followed by the restoration of the oxygen level to normal due to a strong respiratory drive (Somers VK et al. J Clin Invest 1995; 96: 1897-904). Further, the obstructive sleep apnea acts as a cause for cardiovascular complications by secondarily exciting sympathetic nerves and thus promoting adipolysis in fat cells to increase the amount of free fatty acid in the blood (Hucking K. et al. J Clin Invest 2003; 111:257-64), Hypoxic-oxygen restoration promotes the generation of reactive oxygen species (ROS) resulting in an oxidative tissue damage (Schulz R. et al. Am J Respir Crit Care Med 2000;162:566-70) and cytokines such as CRP, IL-6, and TNF-α cause inflammation (Ciftci TU et al. Cytokine 2004;28:87-91). In addition, adipokines such as leptin, adiponectin, and resistin are increased, adhesion molecules such as ICAM-1, VCAM-1, E-selectin, and L-selectin are increased, and endoplasmic reticulum stress is increased by accumulation of unfolded proteins (Tatsumi K et al.Chest 2005; 127: 716-21). As such, the obstructive sleep apnea may accompany cardiovascular complications such as hypertension, cardiac failure, cardiac arrhythmias, ischemic heart disease, stroke, and pulmonary hypertension, and it has been reported that mortality rate is increased statistically significantly in persons whose apnea index (the number of times of apnea per hour) is 20 or more (Shamsuzzaman AS et al. JAMA 2003; 290: 1906-14). Besides, secondary symptoms such as severe daytime lethargy and fatigue due to sleep fragmentation, retrograde memory loss that is a secondary symptom due to excessive sleepiness, decreased attention, decreased judgment, various personality changes (aggressive personality, irritability, anxiety, and depression), erectile dysfunction, and the like maybe developed (Simon S. et al. Chest. 2012 Dec;142(6):1645-51).

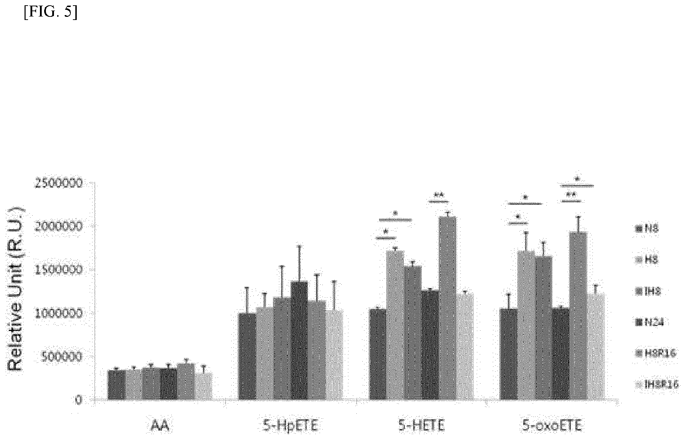

The biomarker of the present disclosure may be used for diagnosing or prognosis of chronic intermittent hypoxia having various symptoms and characteristics and for determining the severity of the disease. For the determination of the severity, the amount of metabolite measured can be correlated with the severity. For example, as shown in FIG. 5, more amounts of the metabolites are detected in the persistent hypoxia.

In the present disclosure, a biological sample or material refers to urine.

In the present disclosure, the term detection or detecting refers to quantitative and/or qualitative analyses. The detection includes a determination of the presence and/or absence as well as the levels of the present markers. The present markers may be detected using the methods known in the art, and the person skilled in the art would be easily able to select appropriate methods for the detection.

The biomarker according to the present disclosure is selected from a 5-hydroxy-6,8,11,14-eicosatetraenoic acid (5-HETE), or a 5-oxo-6,8,11,14-eicosatetraenoic acid (5-oxoETE)The arachidonic acid (AA) is a polyunsaturated fatty acid which is mostly found in cell membranes and decomposed through two main metabolic paths, that is, one is the LO pathway where AA becomes a hydroxyl derivative by lipoxygenase (LO) and the other is the COX pathway where AA is converted to prostaglandin via cycloxygenase (COX).

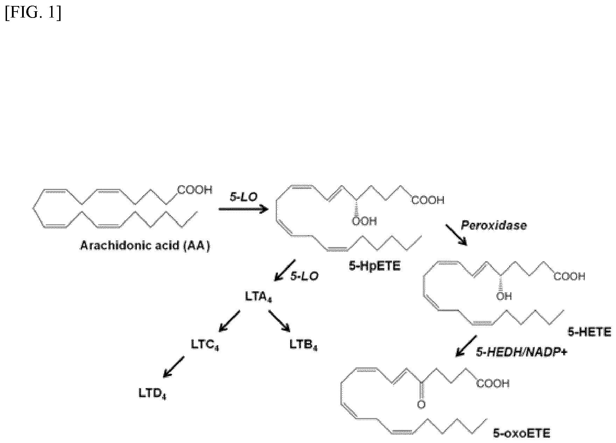

In a reference embodiment of the present disclosure, the composition or the method includes or use a material for detecting an AA metabolite generated particularly in the LO pathway. The LO pathway is schematically depicted in FIG. 1 and as shown in FIG. 1, 5-hydroperoxyeicosatetraeonic acid is an intermediate produced in a process in which an arachidonic acid is generated to leukotriene A4 and may be generated by arachidonate 5-lipoxygenase. 5-hydroxyeicosatetraenoic acid is an intermediate in a biosynthesis of leukotriene and may be generated from 5-hydroperoxyeicosatetraeonic acid by peroxidase as shown in FIG. 1. 5-oxo-6,8,11,14-eicosatetraenoic acid may be generated by oxidation by 5-hydroxyeicosanoid dehydrogenase (5-HEDH).

In the present disclosure, the metabolites via the LO pathway include 5-hydroxy-6,8,11,14-eicosatetraenoic acid (5-HETE), or 5-oxo-6,8,11,14-eicosatetraenoic acid (5-oxoETE).

The AA according to the present disclosure and the metabolic products thereof, that is, the metabolites or the derivatives may be detected by various methods known in the art and appropriate methods may be selected at a level of those skilled in the art. For example, Applied Biochemistry and Biotechnology Jul-Sep 2000, Volume 88, Issue 1-3, pp 33-44; E.J. Want et al. Nature Protocols 2010; 5: 1005 - 1018 and Stanke-Labesque F et al. J Allergy Clin Immunol 2009;124:364-70 maybe referred.

In an exemplary embodiment of the present disclosure, the arachidonic acid derivatives are detected by using a receptor, a ligand, a substrate, an antibody, an antibody fragment, an aptamer, an avidity multimer, or peptidomimetics which specifically recognizes the arachidonic acid derivative of the present disclosure. The detection material may be used in an antibody analysis method, a chemiluminescence analysis method, a liquid chromatography, mass spectrometry method and the like, which are of course, may be used for detecting the biomarker according to the present disclosure.

According to an exemplary embodiment of the present disclosure, the biomarker may be detected by using mass spectrometry which may for example be found in Applied Biochemistry and Biotechnology Jul-Sep 2000, Volume 88, Issue 1-3, pp 33-44.

In another exemplary embodiment, methods employing antibodies may be used. The methods are using materials that specifically recognize the AA derivative thereof of the present disclosure, which include for example polyclonal antibodies, monoclonal antibodies, receptors, ligands, antibody fragments, antibody mimetics, aptamers, avidity multimers, or peptidomimetics. The immunoassays using sandwich system like ELISA (Enzyme Linked Immuno Sorbent Assay), or RIA (Radio Immuno Assay) and the like may be used for quantitative and/or qualitative detection of the present markers. In this system, the biological samples are reacted with a first antibody fixed to a solid substrate/support such as a glass, a plastic (for example, polystyrene), polysaccharides, a bead, a nylon or nitrocellulose membrane or a microplate well to form a complex and the complex is then allowed to react with an second antibody that is usually labeled with agents that can be detected directly or indirectly such as radioactive substances like ³H or ¹²⁵I, fluorescent materials, chemiluminescent substances, hapten, biotin, or digoxygenin and the like. In some cases, the labeling materials are conjugated with an enzyme such as horseradish peroxidase, alkaline phosphatase, or maleate dehydrogenase that is able to produce colors or color changes or illuminate in the presence of appropriate substrates.

Other methods based on immune reaction may also be used. In other embodiment, an Immuno Electrophoresis such as an Ouchterlony plate, a Western blot, a Crossed IE, a Rocket IE, a Fused Rocket IE, or an Affinity IE, which can detect the markers simply by antigen-antibody reaction may be used.

The agents or materials that may be used in the methods described above are known the art. For example, the markers may be detected through an antigen-antibody reaction, or a reaction with a substrate, nucleic acid or peptide aptamers, receptors or ligands that specifically recognize the present markers, or cofactors or using mass spectrometry.

The agents or materials that bind or interact specifically with the markers of the present disclosure can be utilized by means of chip or with nanoparticles. The immunoassay or immunostaining methods as described above are disclosed in the following literatures : Enzyme Immunoassay, E. T. Maggio, ed., CRC Press, Boca Raton, Florida, 1980; Gaastra, W., Enzyme-linked immunosorbent assay(ELISA), in Methods in Molecular Biology, Vol. 1, Walker, J.M. ed., Humana Press, NJ, 1984 etc. The intensities of the signals generated by the immunoassay mentioned above are then analyzed, namely compared with the signals from appropriate controls for the determination whether the sample is related to hypoxia.

In a reference aspect, the present disclosure relates to a kit for diagnosing hypoxic diseases. The kit can be used for diagnosis or prognosis or progression of hypoxia or hopoxia related diseases by quantitative and/or qualitative detection of the present markers, the AA and the metabolite thereof in biological samples of interest.

The kit may be used for the aforementioned various detecting methods. For example, the kits may be formulated as an immunochromatography strip kit, an ELISA kit, a chemiluminescence analysis kit, a luminex kit, and the like. The ELISA kit includes a specific antibody to the biomarker. The antibody employed has a high specificity and affinity to each biomarker and almost no across-reactivity against other biomarkers. Such antibodies are a monoclonal antibody, a polyclonal antibody, or a recombinant antibody. Further, the ELISA kit may include an antibody specific to a control sample. Further, ELISA kits may include reagents capable of detecting the antibody bound to the marker, for example, labelled secondary antibodies, chromophores, enzymes (for example, conjugated to antibodies), and substrates thereof or other materials capable of binding to antibodies.

The luminex kit, as a high-throughput quantitative analysis method capable of simultaneously processing a maximum of 100 different kinds of analytes while employing a small amount (10 to 20 µl) of sample which is not pre-treated, is an analysis method having a high sensitivity (pg unit) and short analysis time, and can replace an existing ELISA or ELISPOT. The luminex assay, as a multiplex fluorescence microplate assay capable of simultaneously processing more than 100 different kinds of biological materials in each well of a 96-well plate, distinguishes and quantifies more than 100 different color groups of polystyrene beads by performing signal transfer in real time by using two kinds of laser detectors. The 100 beads may be constituted to be distinguished in the following ways. At one side, red fluorescence beads are divided into 10 levels or more according to their fluorescent intensity and at the other side, orange fluorescence beads are divided into 10 levels or more, and the beads therebetween shows a unique intensity according to the mixed ratio of the orange and red fluorescence thus generating 100 color-coded bead sets. Further, an antibody specific to the maker to be analyzed is attached to each bead and thus the biomarkers may be quantified by an immune antibody reaction using the beads.

The luminex kit capable of performing the luminex assay includes a specific antibody to the biomarker. The antibody employed has a high specificity and affinity to each biomarker and almost no across-reactivity against other biomarkers. Such antibodies are a monoclonal antibody, a polyclonal antibody, or a recombinant antibody. Further, the luminex kit may also include a specific antibody to a material in a control group. Other luminex kits may also include reagents capable of detecting the antibody bound to the marker, for example, labelled secondary antibodies, chromophores, enzymes (for example, conjugated to antibodies), and substrates thereof or other materials capable of binding to antibodies. The antibody may be an antibody conjugated to microparticles and further, the microparticle may be colored latex or a colloidal gold particle.

In the kit for diagnosing the hypoxic disease or analyzing the prognosis thereof, a kit for diagnosing hypoxic disease including an immunochromatographic strip for diagnosis may be a rapid test or diagnosis kit including essential elements required for performing a rapid test which may show the result within 5 minutes. The immunochromatographic strip may include (a) a sample pad to which a sample is absorbed; (b) a binding pad which is binding to a biomarker in the sample; (c) a reaction membrane in which a reaction line including monoclonal antibodies for the biomarker and a control line are formed; (d) an absorption pad to which the residual sample is absorbed; and (e) a backing or supporting material. The antibody employed has a high specificity and affinity to each biomarker and almost no across-reactivity against other biomarkers. Such antibodies are a monoclonal antibody, a polyclonal antibody, or a recombinant antibody. Further, the rapid test kit may include an antibody specific to a control material. Other rapid test kits may include other materials required for diagnosis such as reagents capable of detecting the bound antibodies, for example, a nitro cellulose membrane to which the specific antibody and the secondary antibody are fixed, a membrane coupled with the beads bound to the antibody, the absorption pad, and the sample pad.

In yet another aspect, the present disclosure further provides a method of detecting a hypoxic marker comprising quantitative and/or qualitative detection of the AA derivative in the biological material from the subject to detect/diagnose chronic intermittent hypoxia, diagnose the hypoxic disease and/or monitor the prognosis of the hypoxia or to provide information required therefor.

The biological samples are separated from the subject and include urine. In the exemplary embodiment of the present disclosure, the urine, particularly, a first morning urine may be used.

Accordingly, the present methods can be advantageously used to diagnose or prognosis chronic intermittent hypoxia or a disease accompanied by chronic intermittent hypoxia by performing qualitative and quantitative analysis of the AA derivative using the methods as described above and comparing the analysis results with an appropriate control group.

According to one exemplary embodiment of the present disclosure, the urine of the hypoxia patient is obtained and the amount of 5-HETE, or 5-oxo ETE in the urine is measured in the samples of each patient by performing a liquid chromatography/mass spectrometer (LC/MS) method, and then the measured value may be used for diagnosis and/or prognosis of chronic intermittent hypoxia in comparison to a control group.

In the present disclosure, the control is a sample from a person who does not suffer from the chronic intermittent hypoxia for example, obstructive sleep apnea in which the controls are divided into a simple snoring group (AHI < 5) and a obstructive sleep apnea group (AHI > 5) based on an apnea-hypopnea Index (AHI), and the simple snoring groups are used as a control. Or apnea patients (for example, primary insomniac patients) other than obstructive sleep apnea who have AHI < 5 and no snoring may also serve as a control group.

As an example, the cut off (upper limit in the case of increasing biomarker/the lower limit in the case of decreasing biomarker) value in a normal range for a particular marker is determined in a control group, which is then used for diagnosis of the subject suspected of the disease and the subject may be diagnosed as hopoxia when the value is increased by about 50% or more compared to cut off value. Particularly early diagnosis of serious hypoxia is possible when the amount of corresponding biomarker is increased by about 2 times or more as compared to cut off value. However, the value is not limited thereto, and may be different depending on the type of a specific material that is used for detecting hypoxia or diagnosing hypoxia-related diseases. For example, when blood or urine is used as a sample, there is high probability the markers are concentrated therein, and thus, the increased value may be large. Thus the values may be determined in consideration of the factors as described above. In addition, by determining whether the amount of corresponding biomarkers in a patient with hypoxia is returned to a normal range after treatment, it is possible to determine and monitoring the efficacy of the therapy used. The diagnosis of chronic intermittent hypoxia using the biomarker according to the present disclosure may be used alone or in combination of other known methods.

Hereinafter, the present disclosure will be described in detail with reference to Examples. However, the scope of the present disclosure is not limited by these Examples.

### Example 1: Selection of biomarker

The biomarkers were selected through analyzing cells (Example 1-1) and samples from the subjects (Example 1-2) as described below.

### Example 1-1: Selection of biomarkers using THP-1 cell line

A THP-1 cell line, a human monocyte-derived cell line was cultured in the conditions of regular oxygen (21% oxygen), low oxygen (1% oxygen), and regular oxygen after low oxygen (reoxygenation), and then, the metabolites included in the culture media were identified and quantified using a LC/Q-TOF MS analysis as follows.

The present Example is to identify the changes in the metabolites in the bodily fluids (including blood and urine) under acute hypoxia condition by exposing the blood cells to various low oxygen conditions

### ① Cell culture

The human-derived monocyte cells (THP-1) (Korean Cell Line Bank, KCLB No. 40202) at 5 × 10⁵ cells/4 mL medium were cultured in DMEM (Dulbecco's modified Eagle's medium) including 10% FBS (fetal bovine serum) under the condition of regular oxygen (21% O₂, 5% CO₂) at 37°C for 24 hours, the condition of low oxygen (1% O₂, 5% CO₂) at 37°C for 24 hours, or the condition of regular oxygen for 16 hours after low oxygen for 8 hours (reoxygenation) at 37°C.

After that, the media without the cells were isolated to obtain the samples to be analyzed. The media were subjected to a centrifugation at 3000 rpm and 4°C to obtain supernatants only. The supernatants were then aliquoted and stored in a -70°C cryogenic deep-freezer, and were thawed immediately before use.

In order to decrease the expression of a hypoxia-inducible factor-1 (HIF-1), a major regulator in a low oxygen environment, two types of si-HIF-lalpha (#1, 5'-CAAAGUUAAAGCAUCAGG-3' ; #2, 5'-UGUACUGUCCUGUGGUGA-3') were prepared, and then used for transfection into the cells using Lipofectamine RNA iMAX reagents (Life Technologies, USA) following the manufacturer's instruction. In addition, 2-methoxyestradiol (2ME2) and 17-N-Allylamino-17-demethoxygeldanamycin (17-AAG) used (Selleck chemicals) as the inhibitors of HIF-1 were used, and YC-1 was purchased from A.G. Scientific. The cells were treated also with 2ME2 and 17-AAG at the concentrations of 100 µM during the exposure of a low oxygen condition.

### ② Preparation of assay sample

H₂O (400 µl) at 4°C and medium (100 µl) were put into a 1.5 ml micro tube so as to be diluted. After that, the sample was mixed well using a vortex,, and then was filtered/centrifuged at 4°C and 14,000 g for 20 minutes to obtain the supernatant thereof. The supernatant was collected in assay vials. In addition, for a QC (quality control) assay, the diluted samples of 50 µl from all the samples were mixed (pooling).

### ③ Analysis of metabolite by LC/Q-TOF MS assay

The samples that were prepared as described above were isolated by the system equipped with a reverse column, Zorbax SB-C18, 50 × 2.1 mm, 1.8 µm (Agilent Technologies, USA) in Binary Agilent 1200 series HPLC (Agilent Technologies). At this time, the assay sample in the amount of 5 µl was injected so as to pass through the column that was heated at 40°C. The metabolite sample was subjected to a gradient elution with 98% solvent A (2 mM ammonium formate and 0.1% formic acid in H₂O) and 2% solvent B (0.1% formic acid in methanol) at the rate of 400 µl/min for 21 minutes. In order to avoid a cross-contamination, a blank run was performed between the sample runs.

ESI algorithm was used by coupling an Agilent 6530 quadrupole time-of-flight (Q-TOF) mass spectrometer (Agilent Technologies) with HPLC. Four centroid data per one second for one spectrum from 100 to 1100 m/z were obtained, and the m/z for all the spectra were calibrated in real time using the reference value externally supplied.

The metabolites were first identified from the mass spectra in the HMDB (http://www.hmdb.ca/) and METLIN (http://metlin.scripps.edu/) database using a software (Agilent MassHunter Qualitative Analysis (version B.05.00), Agilent Mass Profiler Professional (version B.02.02)). After that, the cleavage pattern by the LC/MS/MS assay of the corresponding metabolite was subjected to an additional confirmation work as compared to a practical standard material (Cayman, Ann Arbor, MI, USA).

### ④ Quantitative analysis of metabolite using LC/Q-TOF MS

The relative concentration of the corresponding metabolites was calculated using the chromatograph area of the respective metabolite in the culture solution obtained from the corresponding cells.

The metabolites that were significantly increased in the culture solution subjected to a low oxygen condition as compared to the cell culture solution under a normal oxygen condition were screened, and two metabolites, 5-HETE and 5-oxoETE, were statistically and significantly increased under the condition of low oxygen. For this reason, 5-HETE and 5-oxoETE, and also, AA (reference embodiment) and 5-HpETE (reference embodiment), which were high rank metabolites thereof were selected as a biomarker.

### Example 1-2: Selection of biomarker using subjects to be tested

### Example 1-2-1: Recruitment of subjects to be tested and setting experimental group/control group

The patients in ages of 10 to 60 that visited the otolaryngology clinic in Seoul University Hospital and the Seoul Sleep Center with snoring and sleep apnea syndrome and who agreed to the research were selected as the subjects and subjected to polysomnography. The patients who had a history of tumor or were unsuitable for an operation and polysomnography or could not provide urine due to a kidney disease were excluded. Simple snoring patients (AHI < 5) and the patients with sleep apnea syndrome (AHI > 5) were determined based on an apnea-hypopnea index (AHI) after performing polysomnography. Using the simple snoring patients or insomnia patients as a control group, the results of the experimental group, i.e., the patients with sleep apnea syndrome, were compared and analyzed.

In the present Example, the corresponding metabolite changes were measured in the urine sample of the patient with obstructive sleep apnea syndrome, the average disease period of whom generally extends from several years to several decades to determine the changes in the metabolites in the bodily fluid of the chronic hypoxia.

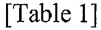

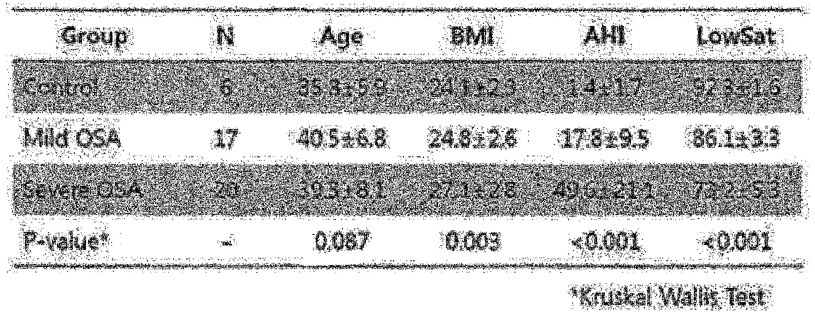

In both the experimental groups and control groups, the basic information of a patient, such as, sex, age, height, body weight, neck size, and waist size was determined, and the results of a basal blood test (CBC, admission panel, and the like) and a radiographic test (cephalometry) which are a test required for patients with sleep apnea syndrome were also collected.

An apnea-hypopnea index (AHI) is an index for determining severity of sleep apnea syndrome, and represented as the numbers of apnea and partial breathing (hypopnea) per hour of sleeping. The patients were classified as normal when the number is 0 to 4, as mild when 5 to 14, as moderate when 15 to 30 and as severe state when over 30.

### Example 1-2-2: Selection of biomarkers

Various indices were collected by performing polysomnography for determine the development and severity of sleep apnea syndrome of the patients and control groups. In addition, the biomarkers that were increased or decreased were screened in the patient groups by identifying and quantifying the metabolites in the first morning urine from the patient groups and control group using a mass spectrometric assay as described below. Finally, AA (reference embodiment), and 5-HpETE (reference embodiment), 5-HETE, and 5-oxoETE were selected as a biomarker based on the correlation between the candidate biomarkers and the major indices obtained from, such as, clinical symptoms and polysomnography.

The correlations among the relative quantitative value which was obtained by calibrating the respective biomarker with the amount of creatine present in the urine, and the lowest oxygen saturation (95% or more as a normal value; the lower value indicates severe sleep apnea syndrome) or AHI (higher value means more severe sleep apnea syndrome) that is an severity index of sleep apnea syndrome determined by polysomnography were analyzed using a Pearson's correlation test as known.

The metabolites included in the urine of the subject to be tested were identified and quantified as described in Example 1-1 using a LC/Q-TOF MS analysis except that the concentration of urine in the metabolite quantitative analysis using LC/Q-TOF MS was calibrated with creatinine. The relative concentration of the metabolite was calculated by dividing the chromatogram area of respective metabolite with the chromatogram area of creatinine.

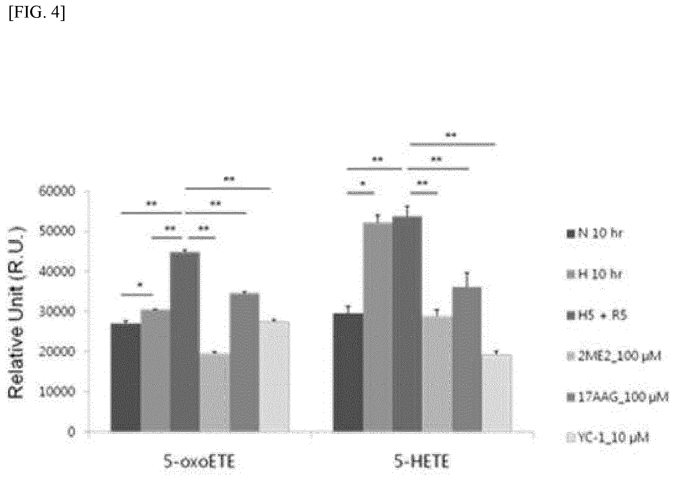

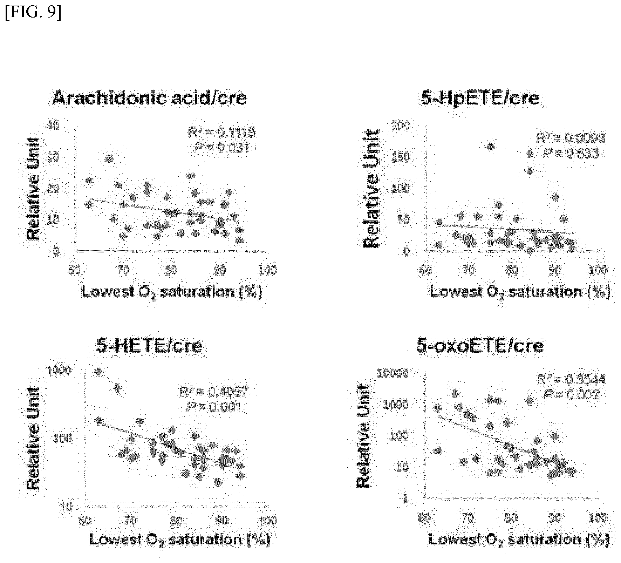

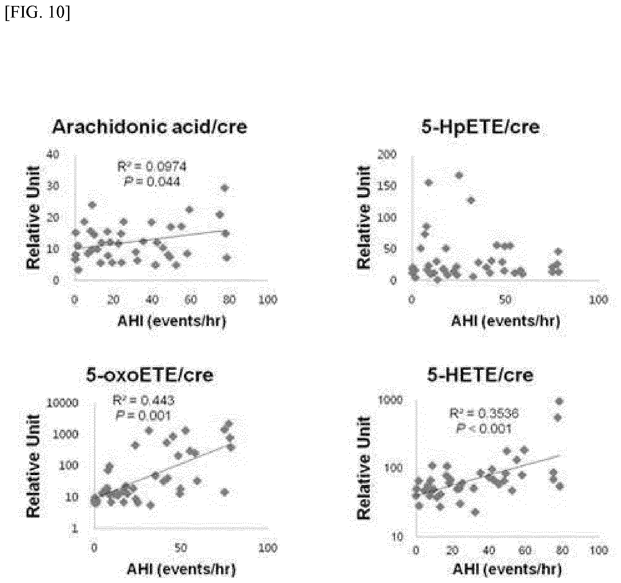

It was confirmed that the arachidonic acid and the derivative thereof were significantly increased in both experiments as described in Examples 1-1 and 1-2 (see FIGs. 1 to 6 and FIGs. 8 to 10).

In detail, it was confirmed that 5-HETE and 5-oxoETE, two types of arachidonate derivatives, were increased in Example 1-1, and an arachidonic acid, 5-HpETE, 5-HETE, and 5-oxoETE were increased in Example 1-2. In the case of Example 1-2, it was thought that since the disease period was generally several years, or 10 years or more, the stimulation by low oxygen was acting for a longer period of time ("chronic"), and thereby, in addition to 5-HETE and 5-oxoETE, an arachidonic acid or a precursor, such as, 5-HpETE were also increased.

The results of Example 1-1 testing the effect of only the low oxygen stimulation to find the biomarkers under the low oxygen condition and Example 1-2 testing the changes of the metabolites in the patient experiencing the low oxygen condition, all support that the present markers can be used advantageously to determine or diagnose hypoxia.

### Example 2: Verification of the biomarkers

### Example 2-1: Verification of the biomarker selected using cell lines

After THP-1 cells were cultured under the conditions of normal oxygen level, low oxygen level, and normal oxygen level after the low oxygen level (reoxygenation) as Example 1-1, AA (reference embodiment), and 5-HpETE (reference embodiment), 5-HETE, and 5-oxoETE in the metabolites included in the culture solutions was quantified using a LC/Q-TOF MS analysis as Example 1.

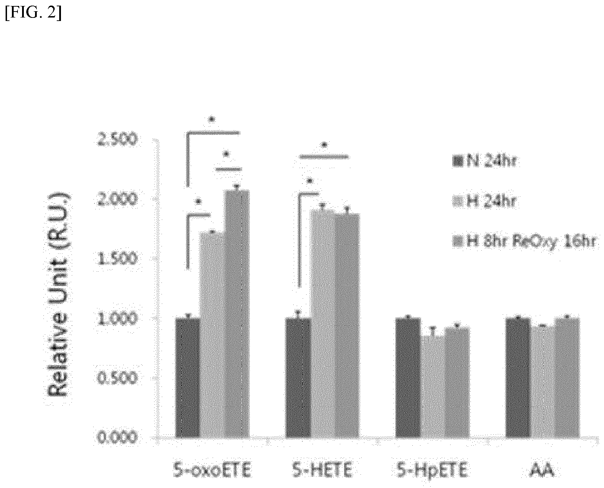

The results are shown in FIG. 2, and all of the markers were metabolites induced or derived from 5-HpETE through an enzymatic function of 5-lipoxygenase (5-LO) and peroxidase in AA and are derivatives of an arachidonic acid (AA).

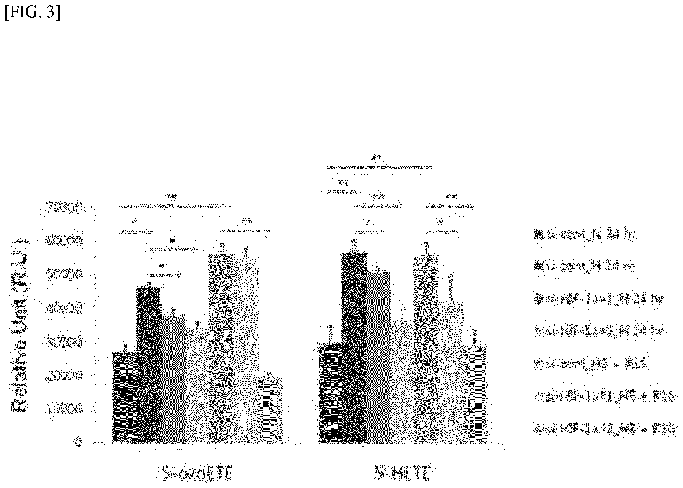

Then, using the method described in Example 1-1, the expression of a hypoxia-inducible factor-1 (HIF-1) that was a major regulator under the low oxygen environment was inhibited using si-RNAs (si-HIF-1a#1 and si-HIF-1a#2), and then, the metabolites were analyzed. The results are shown in FIG. 3. As in FIG. 3, it was confirmed that 5-HETE and 5-oxoETE that were increased under the low oxygen condition (H) were decreased. Similarly, it was confirmed that even when the cells were treated with the inhibitors of HIF-1, 2-methoxyestradiol (2ME2), 17-N-Allylamino-17-demethoxygeldanamycin (17-AAG), and YC-1, 5-HETE and 5-oxoETE that were increased under the low oxygen condition (H) were decreased. It means that 5-HETE and 5-oxoETE that were increased under the low oxygen condition (H) were increased HIF-1-dependently. Especially, it was confirmed that considering that HIF-1 is a major regulator during low oxygen adaptation and metabolic regulation, the increases of 5-HETE and 5-oxoETE under the low oxygen condition reflect the changes that occurs specifically in the body under low oxygen condition. The HIF-dependent increase indicates that the increases of the metabolites according to the present disclosure are closely related to the low oxygen environment.

In addition, the results that were confirmed in THP-1 cells, a human-derived blood cell line, were reconfirmed using a primary human polymorphonuclear cell from PromoCell, Germany. As a cell medium, Mononuclear Cell Medium (C-28030) manufactured by PromoCell was used, and the analysis was performed as described in Example 1. The results are shown in FIG. 5. As in FIG. 5, it was confirmed that as compared to the normal oxygen condition, 5-HETE and 5-oxoETE under the condition of low oxygen for 8 hours and intermittent low oxygen for 8 hours (IH8, the culture was subjected to 1% oxygen condition for 30 minutes, and then, 21% oxygen condition for 30 minutes per hour) were increased.

In addition, it was confirmed that in the case of culturing under the normal oxygen condition for 16 hours after the low oxygen condition for 8 hours (H8R16), 5-HETE and 5-oxoETE were also increased. 5-oxoETE was also increased even in the case of culturing under the normal oxygen condition for 16 hours after the intermittent oxygen condition for 8 hours (IH8R16).

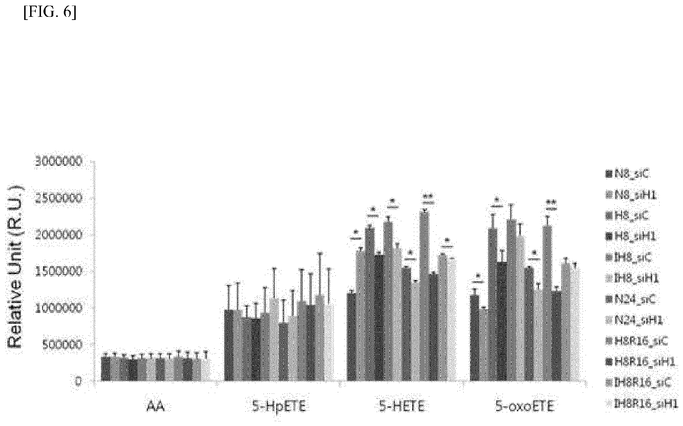

In addition, as shown in FIG. 6, it was confirmed that when HIF-1 alpha in the human primary polymorphonuclear as in THF-1 cells was inhibited by the introduction of si-RNA, 5-HETE and 5-oxoETE that were increased under the low oxygen condition were decreased. These results indicate that the increases of 5-HETE and 5-oxoETE under the low oxygen condition depend on HIF-1.

In the 5-LO metabolic pathway of an arachidonic acid (refer to FIG. 1), considering that the increase starts from 5-HETE under the low oxygen level, it is determined that the process of converting 5-HpETE to 5-HETE was activated under the low oxygen condition. For this reason, the activity of Glutathione Peroxidase (GPX) that is an enzyme for converting 5-HpETE into 5-HETE was measured. The GPX activity was measured using a GPX assay kit (ab102530, Abcam, USA), and the GPX activity measurement principle of the kit was as follows. First, the GPX allows reduced glutathione (GSH) to be converted into oxidized glutathione (GSSG). When the GSSG is again reduced into GSH by glutathione reductase (GR), NADPH was consumed. Then the absorbance at 340 nm is measured using a spectrophotometer so as to measure the level of the decrease of NADPH, which then is used to calculate the activity of GPX.

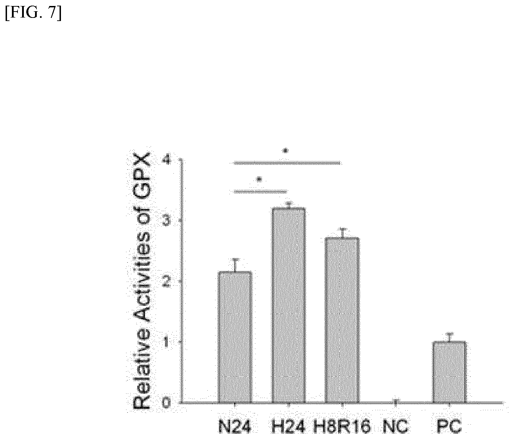

The results are shown in FIG. 7. As in FIG. 7, it was confirmed that when the cells were cultured under the low oxygen condition for 24 hours (H24) and the normal oxygen condition for 16 hours after the low oxygen condition for 8 hours (H8R16), all the activities of GPX were increased.

These results indicate that the hypoxia can be detected or diagnosed by determine the concentrations of an arachidonic acid (reference embodiment), 5-HpETE (reference embodiment), 5-HETE, and 5-oxoETE markers according to the present disclosure.

### Example 2-2: Verification of biomarkers in patient and control group

The metabolites in the patients with obstructive sleep apnea syndrome and the control group were tested using the methods as described in Example 1-2-2, and the correlation of the lowest oxygen saturation collected from polysomnography that was measured at the same day was determined using a Pearson's correlation test. The metabolites were calibrated with creatinine concentration in the urine, and quantified.

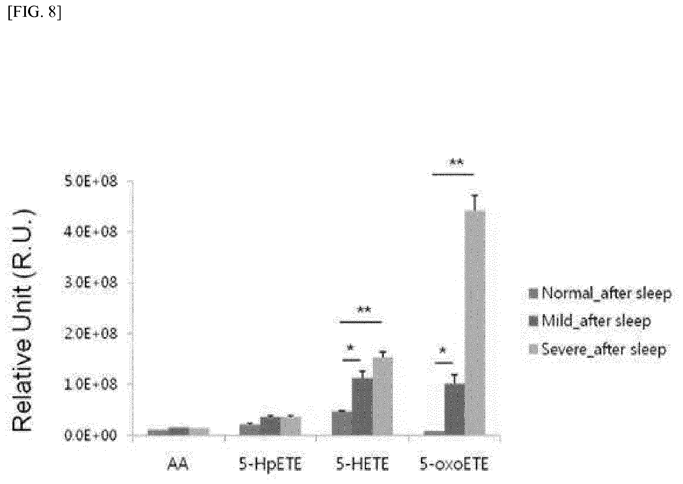

As a result in FIGs. 8 to 10, it was confirmed that the lower the oxygen saturations levels are, the higher the level of all the markers, i.e., an arachidonic acid, 5-HpETE, 5-HETE, and 5-oxoETE metabolites are. Especially, 5-HETE and 5-oxoETE showed a close correlation with the lowest oxygen saturation. This indicates that 5-HETE and 5-oxoETE are increased more in severe sleep apnea syndrome patient who are exposed under a low oxygen condition for a longer period of time than the less severe patient.

In other words, it was confirmed that as the metabolisms are progressed, arachidonic acid (reference embodiment), 5-HpETE (reference embodiment), 5-HETE, and, 5-oxoETE in the urine of the patient with obstructive sleep apnea syndrome were detected in a higher amount. Especially, it was confirmed that 5-HETE and 5-oxoETE in the urine of the patient with severe sleep apnea syndrome were detected in a higher amount as compared to the patient with mild sleep apnea syndrome, and thus can be used to determine the severity of a disease.

Data were represented as an average and standard deviation were used, the difference between different tissues from the same patient was verified using a Wilcoxon signed rank test, a non-parametric testing method, and the differences between the experimental group and control group were analyzed using a Mann-Whitney test. The correlations between the selected biomarkers and the conventional major disease indices were analyzed, and if necessary, a layering analysis was performed.

## Claims

1. An *in vitro* method for detecting chronic intermittent hypoxia, the method comprising:
detecting an arachidonic acid derivative selected from a 5-hydroxy-6,8,11,14-eicosatetraenoic acid (5-HETE), or a 5-oxo-6,8,11,14-eicosatetraenoic acid (5-oxoETE) from a biological sample: and
diagnosing the sample as chronic intermittent hypoxia when the amount of the arachidonic acid derivative in the biological sample is increased as compared to a control group, wherein the biological sample is urine.

2. The method of claim 1, wherein the detection is performed by an antibody analysis, a chemiluminescent assay, or a liquid chromatography/mass spectrometry assay.

3. Use of an arachidonic acid derivative selected from a 5-hydroxy-6,8,11,14-eicosatetraenoic acid (5-HETE), or a 5-oxo-6,8,11,14-eicosatetraenoic acid (5-oxoETE) for the *in vitro* detection of chronic intermittent hypoxia.

## Patentansprüche

1. In-vitro-Verfahren zum Detektieren von chronischer intermittierender Hypoxie, wobei das Verfahren umfasst:
Detektieren eines Arachidonsäurederivats, das aus einer 5-Hydroxy-6,8,11,14-Eicosatetraensäure (5-HETE) oder einer 5-Oxo-6,8,11,14-Eicosatetraensäure (5-oxoETE) ausgewählt ist, aus einer biologischen Probe; und
Diagnostizieren der Probe als chronische intermittierende Hypoxie, wenn die Menge des Arachidonsäurederivats in der biologischen Probe im Vergleich zu einer Kontrollgruppe erhöht ist, wobei die biologische Probe Urin ist.

2. Verfahren nach Anspruch 1, wobei die Detektion durch eine Antikörperanalyse, einen Chemilumineszenztest oder einen Flüssigchromatographie/Massenspektrometrie-Test durchgeführt wird.

3. Verwendung eines Arachidonsäurederivats, das aus einer 5-Hydroxy-6,8,11,14-Eicosatetraensäure (5-HETE) oder einer 5-Oxo-6,8,11,14-Eicosatetraensäure (5-oxoETE) ausgewählt ist, für die In-vitro-Detektion von chronischer intermittierender Hypoxie.

## Revendications

1. Procédé *in vitro* de détection d'une hypoxie intermittente chronique, le procédé comprenant :
la détection d'un dérivé de l'acide arachidonique sélectionné parmi un acide 5-hydroxy-6,8,11,14-éicosatétraénoïque (5-HETE), ou un acide 5-oxo-6,8,11,14-éicosatétraénoïque (5-oxoETE) dans un échantillon biologique ; et
le diagnostic de l'échantillon comme hypoxie intermittente chronique lorsque la quantité du dérivé d'acide arachidonique dans l'échantillon biologique est accrue comparé à un groupe témoin, dans lequel l'échantillon biologique est l'urine.

2. Procédé selon la revendication 1, dans lequel la détection est réalisée par une analyse d'anticorps, un dosage par chimiluminescence, ou un dosage par chromatographie en phase liquide/spectrométrie de masse.

3. Utilisation d'un dérivé d'acide arachidonique sélectionné parmi un acide 5-hydroxy-6,8,11,14-éicosatétraénoïque (5-HETE), ou un acide 5-oxo-6,8,11,14-éicosatétraénoïque (5-oxoETE) pour la détection *in vitro* de l'hypoxie intermittente chronique.
